Europäisches Patentamt

European Patent Office

Office européen des brevets

(11) Veröffentlichungsnummer: **0 338 186**
**A1**

## (12) EUROPÄISCHE PATENTANMELDUNG

(21) Anmeldenummer: **89100382.4**

(22) Anmeldetag: **11.01.89**

(51) Int. Cl.⁴: **A61F 13/14**

(30) Priorität: **22.04.88 DE 8805333 U**

(43) Veröffentlichungstag der Anmeldung:
**25.10.89 Patentblatt 89/43**

(84) Benannte Vertragsstaaten:
**AT BE CH DE ES FR IT LI NL SE**

(71) Anmelder: **FRANZ KALFF GMBH**

**D-5350 Euskirchen(DE)**

(72) Erfinder: **Kalff, Friedhelm**
**Sternenstrasse 3**
**D-5350 Euskirchen(DE)**

(74) Vertreter: **Selting, Günther, Dipl.-Ing. et al**
**Patentanwälte von Kreisler, Selting, Werner**
**Deichmannhaus am Hauptbahnhof**
**D-5000 Köln 1(DE)**

(54) **Saugeinlage für Still-Büstenhalter.**

(57) Die Saugeinlage weist eine runde Saugscheibe (10) auf, die an einer Seite mit einem Klebepunkt (16) versehen ist. Der Klebepunkt (16) ist mit einer Schutzfolie (15) bedeckt. Nachdem die Schutzfolie abgezogen wurde, wird die Saugeinlage an der Innenseite des Körbchens eines Büstenhalters befestigt.

FIG.1

Xerox Copy Centre

EP 0 338 186 A1

## Saugeinlage für Still-Büstenhalter

Die Erfindung betrifft eine Saugeinlage nach dem Oberbegriff des Anspruchs 1.

Die bekannten Saugeinlagen für Still-Büstenhalter haben den Nachteil, daß sie leicht verrutschen und nicht permanent fixiert werden können.

Der vorliegenden Erfindung liegt die Aufgabe zugrunde, eine Saugeinlage der im Oberbegriff des Anspruchs 1 angegebenen Art zu schaffen, die problemlos fixiert und andererseits auch ohne Schwierigkeiten aus dem Büstenhalter entfernt werden kann.

Die Lösung dieser Aufgabe erfolgt erfindungsgemäß mit den im kennzeichnenden Teil des Anspruchs 1 angegebenen Merkmalen.

Die erfindungsgemäße Saugeinlage besteht aus einer im wesentlichen runden Saugscheibe. Diese ist vorzugsweise kreisrund, kann aber auch oval sein oder auf andere Weise von der Rundform abweichen. Der Scheibendurchmesser liegt etwa in der Größenordnung von 10 cm, genauer gesagt zwischen 8 und 11 mm. Diese Scheibe weist an einer Seite ein Haftelement auf, mit dem sie an der Innenseite des Körbchens des Büstenhalters befestigt wird, nachdem zuvor die Schutzfolie abgezogen wurde. Das Haftelement kann ein Klebepunkt sein, vorzugsweise aus einem ausgestanzten Stück Doppelklebeband. Er haftet fest an der Scheibe und kann mit seiner Außenseite an dem Textilmaterial des Büstenhalters befestigt werden. Zum Entfernen der Saugeinlage braucht die Scheibe lediglich abgezogen zu werden, wobei sich der Klebepunkt von dem Textilmaterial des Büstenhalters löst, während er an der Scheibe haften bleibt, die anschließend fortgeworfen wird. Das Haftelement kann auch aus dem einen Teil eines Klettenverschlusses bestehen, dessen anderes Teil im Körbchen des Büstenhalters befestigt ist.

Anstelle des einen Haftelements können auch mehrere Haftelement vorhanden sein.

Vorzugsweise ist ein Haftelement im Zentrum der Scheibe angeordnet. An dieser Stelle kann das Haftelement die Saugscheibe am besten an ihrem vorgesehenen Platz halten, wobei geringfügige Drehungen der Saugscheibe um das Zentrum herum möglich sind.

Bei einer anderen Ausführungsform ist das Haftelement außermittig angeordnet, etwa in der Mitte eines Radius der Scheibe.

Die Saugscheibe besteht z.B. aus Zellstoff, Zellulose, Watte oder einer Mischung daraus.

Im folgenden werden unter Bezugnahme auf die Zeichnungen Ausführungsbeispiele der Erfindung näher erläutert.

Es zeigen:

Fig. 1 eine Ansicht einer ersten Ausführungsform der Saugeinlage,

Fig. 2 einen Querschnitt durch die Saugeinlage nach Fig. 1, und

Fig. 3 eine Ansicht einer zweiten Ausführungsform.

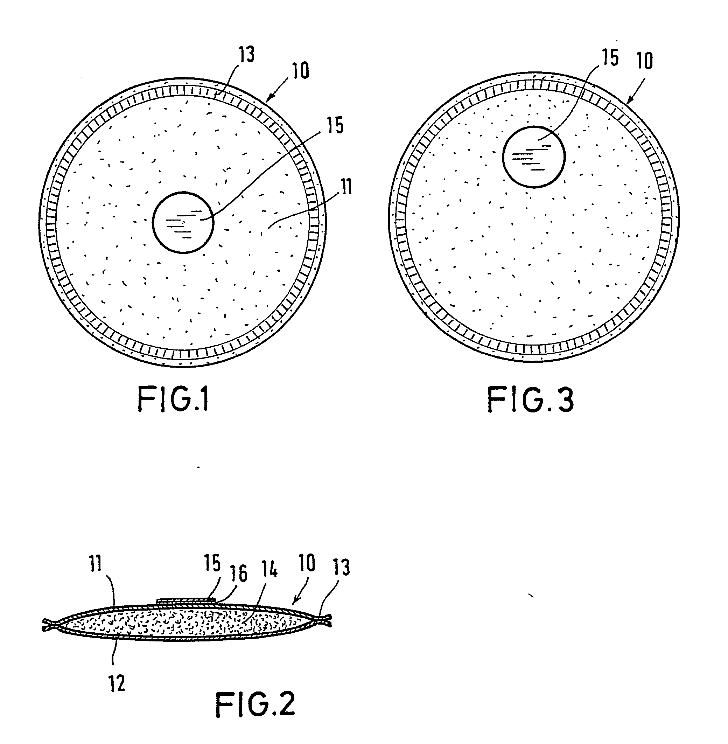

Die Saugeinlage der Fign. 1 und 2 besteht aus einer kreisrunden Saugscheibe 10, z.B. aus Zellulose, mit einem Durchmesser von etwa 10 cm. Die Saugscheibe 10 weist zwei Vliesstoffbahnen 11,12 auf, die an dem umlaufenden Rand durch eine Druckriffelung 13 miteinander verbunden sind. Im Inneren des von den Bahnen 11,12 und der Druckriffelung 13 umschlossenen Raums befindet sich eine saugfähige Wattefüllung 14, die gleichzeitig eine größere Weichheit der Saugeinlage bewirkt.

Im Zentrum der Saugscheibe 10 ist auf der einen Bahn 11 ein mit einer Schutzfolie 15 bedeckter Klebepunkt 16 angeordnet. Dieser Klebepunkt 16 besteht aus einer Doppelklebefolie, die auf beiden Seiten mit Klebemittel beschichtet ist. Die eine Seite ist an die Bahn 11 fest angeklebt und die andere Seite ist mit der Schutzfolie 15 aus Silikonpapier bedeckt. Die Schutzfolie 15 kann abgezogen werden, um die äußere Klebeseite des Klebepunkts 16 freizulegen.

· Die Saugeinlage wird in das Körbchen eines Still-Büstenhalters eingelegt, nachdem die Schutzfolie abgezogen wurde, und sie wird an dem Textilmaterial des Büstenhalters mit dem Klebepunkt 16 fixiert.

Das Ausführungsbeispiel von Fig. 3 unterscheidet sich vom ersten Ausführungsbeispiel nur dadurch, daß der Klebepunkt 16 mit der Schutzfolie 15 nicht im Zentrum der Saugscheibe angeordnet ist, sondern außermittig. Bei dem vorliegenden Beispiel ist der Klebepunkt etwa auf der Hälfte eines Radius der Saugscheibe 10 angeordnet.

## Ansprüche

1. Saugeinlage für einen Still-Büstenhalter, **dadurch gekennzeichnet,** daß eine im wesentlichen runde Saugscheibe (10) an einer Seite mit mindestens einem Haftelement versehen ist.

2. Saugeinlage nach Anspruch 1, dadurch gekennzeichnet, daß das Haftelement ein mit einer abreißbaren Schutzfolie bedeckter Klebepunkt (16) ist.

3. Saugeinlage nach Anspruch 1 oder 2, dadurch gekennzeichnet, daß ein Haftelement im Zentrum der Scheibe (10) angeordnet ist.

4. Saugeinlage nach einem der Ansprüche 1 bis 3, dadurch gekennzeichnet, daß ein Haftelement etwa in der Mitte eines Radius der Scheibe (10) angeordnet ist.

5. Saugeinlage nach Anspruch 1, dadurch gekennzeichnet, daß das Haftelement aus dem einen Teil eines Klettenverschlusses besteht.

6. Saugeinlage nach einem der Ansprüche 1 bis 5, dadurch gekennzeichnet, daß die Saugscheibe (10) zwei an ihrem Rand umlaufend verbundene Bahnen (11,12) und zwischen diesen eine Wattefüllung (14) aufweist.

FIG.1

FIG.3

FIG.2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | US-A-4 674 510 (SNEIDER) <br> * Zusammenfassung; Figuren * | 1-4,6 | A 61 F 13/14 |
| Y | | 5 | |
| Y | DE-A-3 139 808 (RITZENHOFF) <br> * Seite 8, Zeilen 12-19; Figuren * <br> --- | 5 | |
| A | DE-A-3 311 484 (NUSSBAUM) <br> * Figuren * <br> --- | 4 | |
| A | FR-A-1 406 005 (ROSY) <br> --- | | |
| A | EP-A-0 214 867 (JOHNSON & JOHNSON) <br> ----- | | |

**RECHERCHIERTE SACHGEBIETE (Int. Cl.4)**

A 61 F
A 41 C

**Der vorliegende Recherchenbericht wurde für alle Patentansprüche erstellt**

| Recherchenort | Abschlußdatum der Recherche | Prüfer |
|---|---|---|
| DEN HAAG | 14-07-1989 | STEENBAKKER J. |

EPO FORM 1503 03.82 (P0403)

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus andern Gründen angeführtes Dokument
........................................................................
& : Mitglied der gleichen Patentfamilie, übereinstimmendes
    Dokument